# EUROPÄISCHE PATENTSCHRIFT

(11) **EP 2 344 247 B1**
(45) Veröffentlichungstag und Bekanntmachung des Hinweises auf die Patenterteilung: **17.10.2012**
(21) Anmeldenummer: 09737777.4
(22) Anmeldetag: 19.03.2009
(51) Int. Cl.: A61N 2/02, A61B 17/86, H01F 5/00

(54) **VORRICHTUNG ZUM STIMULIEREN EINES HEILUNGSPROZESSES IM BEREICH EINES IMPLANTATS**
APPARATUS FOR STIMULATING A HEALING PROCESS IN THE REGION OF AN IMPLANT
DISPOSITIF POUR STIMULER UN PROCESSUS DE GUÉRISON DANS LA ZONE D'UN IMPLANT

(30) Priorität: 30.04.2008 DE 102008021574
(43) Veröffentlichungstag der Anmeldung: 20.07.2011
(73) Patentinhaber: Neue Magnetodyn GmbH, 80333 München (DE)
(72) Erfinder: KRAUS, Werner, 80539 München (DE); KRAUS, Stephanie, 80538 München (DE); STEPHAN, Heribert, 80689 München (DE)
(74) Vertreter: Schumacher & Willsau
(86) Internationale Anmeldenummer: PCT/EP2009/002048
(87) Internationale Veröffentlichungsnummer: WO 2009/132733

(56) Entgegenhaltungen:
- EP-A2- 2 074 958
- DE-A1-102006 018 191
- US-A1- 2005 012 617
- US-A1- 2005 247 319

## Beschreibung

Die Erfindung betrifft eine Vorrichtung zum Stimulieren eines Heilungsprozesses im Bereich eines Implantats.

Gemäß der Technik nach Kraus und Lechner ist es bekannt, in die Bestandteile von Stützmetall-Osteosyntheseeinrichtungen oder Gelenkendoprothesen Spulenanordnungen, die als Aufnehmer- oder Übertragerspulen bezeichnet werden, zu integrieren und deren Pole mit als Elektroden wirkenden Implantatabschnitten elektrisch zu verbinden. In dieser Anordnung wird im Kernfeld einer externen Magnetspule in Verbindung mit einem Funktionsstromgenerator eine Spannung induziert, die den Heilprozess wirksam unterstützt.

Ein Beispiel für eine Osteosyntheseeinrichtung, die von der geschilderten Technik Gebrauch macht, ist in der DE 10 2006 018 191 A1 offenbart. Die in der DE 10 2004 024 473 A1 beschriebenen Hüftkopfkappenimplantate sind Beispiele für den Einsatz der Technik in der Gelenkendoprothetik.

Bei der Anwendung der geschilderten Technik sind in der Praxis insbesondere die nachfolgend diskutierten Aspekte zu berücksichtigen.

Ein erster Aspekt betrifft die räumliche Zuordnung der Achsen des induzierenden Magnetspulenfeldes zu der Achse beziehungsweise der von dem induzierenden Magnetfeld wirksam durchfluteten Fläche der Aufnehmerspule. Bei den Anwendungen der Elektro-Osteosynthese und der Elektro-Gelenkendoprothese wird die Aufnehmerspule durch Einkleben beziehungsweise Einschieben in beispielsweise die Bohrung kanülierter Knochenschrauben oder durch Einschrauben der die Aufnehmerspule enthaltenden Endkappe eines Knochenmarknagels zu einem mechanisch fest fixierten Bestandteil des Implantates. Die Verankerung des Implantats im Knochen wird aber in erster Linie von den Anforderungen optimaler Stützfunktion seiner Fragmente bestimmt. Daraus folgt für die Wicklungsachse der Aufnehmerspule im Knochen-Stützimplantat eine Variationsbreite ihres Raumwinkels zur langen Knochen- beziehungsweise Körperachse von 0 bis 90°. Dagegen ist der Richtwinkel des induzierenden Magnetfeldes in einer externen Magnetspule relativ begrenzt: Bei der Solenoid-Form, wobei insbesondere Extremitäten im Inneren einer Solenoid-Spule gelagert werden, verläuft der Vektor des induzierenden Spulenkernfeldes parallel zur langen Knochenachse. Bei einer Anordnung nach Heimholtz, wenn Körperteile in dem Zwischenraum eines Helmholtzspulenpaares gelagert werden, dagegen senkrecht dazu. Für die Aufnehmerspule in einer Osteosynthese-Schraube, beispielsweise zur Fixierung einer Schrägfraktur, wird im ungünstigsten Falle ein Winkel zwischen dem Magnetfeld der induzierenden Solenoid- oder Helmholtz-Spule und der Aufnehmerspule des Implantates von 45° erreicht. Dadurch entsteht ein Verlust der induzierten elektrischen Spannung von rund 30 %. Ist das System also bei optimalen geometrischen Verhältnissen auf den für die Knochenstimulation erforderlichen Sollwert von 700 mV ausgelegt, so werden in der geschilderten suboptimalen jedoch praxisnahen Situation nur noch knapp 500 mV erreicht. Im Zusammenhang mit der Endoprothetik liegen vergleichbare Verhältnisse vor.

Ein zweiter Aspekt betrifft die Frequenz der Schwingungen des induzierenden Magnetfeldes, die einen weiteren wichtigen biologischen Wirkfaktor darstellt. Es zeigt sich, dass die für die Aktivierung von Bindegewebe- und Knochenzellen relevanten Botenstoffe Ca²⁺ und cAMP einer Resonanz von Schwingungen ihrer Dichte bei 15 Hz folgen. Deshalb soll der Frequenzbereich von 1 bis 30 Hz, vorzugsweise 10 bis 20 Hz, der elektromagnetischen Induktion beibehalten werden, so dass die Frequenz der Schwingungen kaum als Parameter zur Beeinflussung der Therapiesituation zur Verfügung steht.

Ein dritter Aspekt betrifft die Signalform des induzierenden Magnetfeldes, die den zellulären Wirkfaktor des zeitlichen Verlaufs der Magnetflussdichte in der erkrankten Körperregion bestimmt. Die optimale stimulative Wirkung auf die kompetenten Zellen (Fibroblasten und Osteoblasten) bezüglich ihrer Differenzierung, ihres Stoffwechsels und ihrer Synthese von Strukturproteinen wurde durch sinuide Verlaufsformen erreicht. Sie zeigen eine beträchtliche Überlegenheit bezüglich ihres Effektivwertes und der Impedanz im Vergleich zu den Wirkungen anderer Systeme, deren Induktion mit Nadelimpuls-Bursts oder Rechteckschwingungen mit höhergradigen Oberwellen über 100 kHz und ausgeprägten Unstetigkeiten (Diskontinuitäten) ihrer zeitlichen Verlaufsformen erreicht wird.

Der Erfindung liegt die Aufgabe zugrunde, die Technik der magnetisch induzierten Elektro-Osteo-Stimulation in verbesserter Weise so weiterzubilden, dass die induzierte Spannung die physiologisch erwünschte Sollspannung auch bei suboptimalen geometrischen Verhältnissen erreicht, wobei in diesem Zusammenhang eine Erhöhung der erwünschen magnetischen Flussdichte ausscheidet, dass für die erregende Schwingung weiterhin die Resonanzfrequenz zellulärer Botenstoffe (Ca²⁺ und cAMP) von 15 Hz innerhalb eines klinisch erprobten Frequenzspektrums von 2 bis 30 Hz eingehalten wird und dass die Schwingungs- oder Signalform des induzierenden elektromagnetischen Feldes frei von Unstetigkeiten (Diskontinuitäten) kontinuierlich verläuft.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung besteht in einer Vorrichtung zum Stimulieren eines Heilungsprozesses mit einer mit einem Funktionsstromgenerator gekoppelten Spulenanordnung zum Erzeugen eines elektromagnetischen Feldes in einem erkrankten Körperbereich, einer Steuereinheit zum Beeinflussen eines von dem Funktionsstromgenerator erzeugten Spannungsverlaufs in Abhängigkeit von an eine Eingangsschnittstelle der Steuereinheit übertragenen Signalen, mindestens einem in dem erkrankten Körperbereich angeordneten Implantat, das mit einer Übertragerspulenanordnung gekoppelt ist, wobei die Pole der Übertragerspulenanordnung mit einem Elektrodenpaar verbunden sind, einer Erfassungseinrichtung zum Erfassen einer zwischen den Elektroden des Elektrodenpaars vorliegenden elektrischen Spannung, einer Übertragungseinrichtung zum Übertragen von für die elektrische Spannung charakteristischen Signalen an die Steuereinheit. Auf diese Weise gelingt es, auf eine unter den gewünschten Spannungswert absinkende Induktionsspannung zu reagieren und durch Beeinflussung des von dem Funktionsgenerator erzeugten Spannungsverlauf die gewünschte Spannung letztlich einzustellen. Mitunter kann dies durch eine geringfügige Veränderung des Spannungsverlaufs unter sonstiger Aufrechterhaltung der Magnetfeldcharakteristika erreicht werden, so dass keine Flussdichtenerhöhung und keine Änderung der Grundfrequenz erforderlich ist. Die erfindungsgemäße Vorrichtung ist unter anderem durch eine Erfassungseinrichtung und eine Übertragungseinrichtung gekennzeichnet. Im Spezialfall können diese Einrichtungen miteinander integriert beziehungsweise identisch sein.

Es ist bevorzugt, dass die Übertragungseinrichtung mindestens einen Sender zur drahtlosen Kommunikation mit einem der Eingangsschnittstelle der Steuereinheit zugehörigen Empfänger aufweist. Ein solcher Sender kann in verschiedener Weise ausgestaltet sein. Wesentlich ist, dass er die von der Erfassungseinrichtung erfassten Messwerte in der Weise umsetzt, dass entsprechende Signale an den Empfänger der Steuereinheit übermittelt werden können. Eine Möglichkeit einer solchen Informationsübertragung vom Sender zum Empfänger in der Steuereinheit besteht in der aktiven Erzeugung von Sendesignalen, die von den Messwerten der Sensoranordnung abhängen.

Es kann aber auch vorgesehen sein, dass die Übertragungseinrichtung mindestens einen RFID-Transponder aufweist, dessen Informationsgehalt von einem der Eingangsschnittstelle der Steuereinheit zugehörigen Lesegerät erfassbar ist. Bei einem RFID-Transponder handelt es sich um eine Einrichtung, die nur durch ihr Zusammenspiel mit einem Lesegerät Informationen "aussenden" kann. Letztlich empfängt der RFID-Transponder zu diesem Zweck ein elektromagnetisches Hochfrequenzfeld, das von dem Lesegerät erzeugt wird, um dieses dann in Abhängigkeit von in dem RFID-Transponder gespeicherten Informationen zu verändern. Diese Veränderung wird vom Lesegerät erfasst. Aufgrund dieser im Vergleich zu herkömmlichen aktiven Sendern sehr eingeschränkten Funktionalität eines RFID-Transponders, ist dieser kostengünstig und platzsparend.

Die Informationsübertragung von dem RFID-Transponder zu dem Lesegerät kann auf der Grundlage stattfinden, dass der auslesbare Informationsgehalt des RFID-Transponders in Abhängigkeit von Signalen, die von der Sensoranordnung geliefert werden, veränderbar ist. Im einfachsten Fall werden von der Erfassungseinrichtung verschiedene Spannungen an den Speicher des RFID-Transponders angelegt, wobei diese Spannungen von der von der Erfassungseinrichtung erfassten Spannung abhängen oder die erfasste Spannung selbst ist; im letzten Fall sind Erfassungseinrichtung und Übertragungseinrichtung als miteinander integriert oder identisch zu bezeichnen. Verschiedene Spannungen können nun bewirken, dass der Speicherinhalt des RFID-Transponders verändert wird, so dass letztlich auch die von dem RFID-Transponder an das Lesegerät übertragene Kennung geändert wird. Damit die Möglichkeit besteht, den Speicherinhalt des RFID-Transponders zu verändern, ist es erforderlich, beschreibbare RFID-Transponder zu verwenden.

Alternativ oder zusätzlich ist es aber auch möglich, dass mehrere RFID-Transponder vorgesehen sind, die in Abhängigkeit von Signalen, die von der Sensoranordnung geliefert werden, aktivierbar oder deaktivierbar sind. In diesem Fall sind nicht beschreibbare Transponder ausreichend. Eine oder mehrere Schwellenwertschaltungen, in die die Erfassungseinrichtung und die RFID-Transponder eingebunden sind, sorgen dafür, dass in Abhängigkeit der gelieferten Spannungen unterschiedliche RFID-Transponder aktiv beziehungsweise inaktiv sind. Auch auf diese Weise kann das Lesegerät also unterschiedliche Kennungen je nach Spannung empfangen, also auch auf dieser Grundlage dafür sorgen, dass der Funktionsstromgenerator einen an die erfassten Eigenschaften des erkrankten Körperbereiches angepassten Spannungsverlauf erzeugt.

Die Erfindung ist in besonders vorteilhafter Weise dadurch weitergebildet, dass die Spulenanordnung eine Spule mit einer einen Kreuzungspunkt aufweisenden Spulenwicklung aufweist, die durch eine achtförmige Gestalt zwei Flächen definiert, wobei die Flächen so zueinander ausgerichtet sind, dass die von einem durch einen Stromfluss in der Spulenanordnung erzeugten, die Flächen durchdringenden Magnetfelder im Wesentlichen gleichgerichtet sind. Die Wirkung von zwei getrennten Induktionsspulen mit gleicher Richtung ihres Magnetfeldes, in dem die lädierte Körperregion gelagert wird, kann auf diese Weise auch mit einer einzigen Spule erreicht werden. Dies macht die Anwendung komfortabel, insbesondere aufgrund des verminderten apparativen Aufwands.

Es ist besonders nützlich, dass die Spulenanordnung flexibel ist, so dass die Flächen auf gegenüberliegenden Seiten des erkrankten Körperbereichs angeordnet werden können. Durch ihre flexible Wicklung, die insbesondere durch eine Elastizität des verwendeten Materials bedingt sein kann, wird die Umformung im Sinne eines Achter- oder Unendlich-Zeichens ermöglicht. Die sich durch diese Umformung der Spulenwicklung ergebenden "Schlaufen" der Spule können beispielsweise an beiden Seiten einer Extremität angelegt werden. Sie können je nach Anwendung gleiche oder unterschiedliche Größen haben. Die Spulenanordnung kann durch Ihre Flexibilität sehr vielseitig eingesetzt werden. Im Zusammenhang mit der Auslegung der Spulenanordnung kann vorgesehen sein, dass an zwei dem Kreuzungspunkt abgewandten Positionen der Spulenanordnung zusammenwirkende Befestigungsmittel vorgesehen sind, durch die die Ausrichtung der Flächen zueinander geschaffen und aufrechterhalten werden kann. Die Befestigungsmittel können beispielsweise mittels Gurten, Druckknöpfen, Klettverschlüssen, Schnallen und dergleichen realisiert sein. In besonders vorteilhafter Weise kann vorgesehen sein, dass der Kreuzungspunkt der Spulenanordnung durch eine Kopplungseinrichtung fixierbar ist. Eine solche Kopplungseinrichtung kann beispielsweise durch ein elastisches Band mit Klettverschluss oder Gürtelschließe realisiert sein. Es ist von besonderem Vorteil, dass die Lage des Kreuzungspunktes und somit die Maße der Flächen variabel sind. Durch das auf diese Weise wählbare Flächenverhältnis der Spulenschlaufen ist die magnetische Induktionsflussdichte, die als Quotient aus dem magnetischen Fluss und der betrachteten Fläche definiert ist, einstellbar. Das Verhältnis der Induktionsflussdichten ist der Kehrwert des Verhältnisses der jeweils zugehörigen Flächen. Das Flächenverhältnis der beiden Schlaufen kann nach den therapeutischen Erfordernissen gewählt werden, indem eine veränderbare Fixierung des Kreuzungspunktes durch variable Anordnung der Kopplungseinrichtung ermöglicht wird. Beispielsweise kann in einem Zielbereich des Körpers eine hohe magnetische Induktionsflussdichte dadurch erzielt werden, dass eine kleinflächige Spulenschlaufe in dessen Nähe gebracht wird, während im Hinblick auf die andere großflächige Spulenfläche zur Bereitstellung des Helmholtzspuleneffektes hauptsächlich auf deren parallele Anordnung zur kleineren Fläche zu achten ist. Mit einer solchen Spulenanordnung wird erreicht, dass das Magnetfeld der beiden sich gegenüberstehenden Schlaufen der Spulenanordnung durch die räumliche Umkehr der Stromrichtung in einer der beiden Schlaufen, wie bei der Anordnung von zwei getrennten Spulen nach Heimholtz, gleichgerichtet ist, wobei eine besonders gute und flexible Handhabbarkeit zur Verfügung gestellt wird. Eine einfache Handhabung bei der Anpassung der geometrischen Form der Spulenanordnung an die Lage der jeweiligen Knochen beziehungsweise Weichgewebeläsion wird ermöglicht. Beispielsweise können die Schlaufenflächen an Behandlungsbereiche wie Fuß, Knie Unterschenkel, Oberschenkel, Becken, Wrbelsäule, Hand, Unterarm, Oberarm, Kiefer und Schädelbereich angepasst werden; durch Variation der Schlaufenformen und - größen auch im Hinblick auf die Stärke des Magnetfeldes. Eine weitere Eigenschaft der erfindungsgemäßen Spulenanordnung ist eine Zunahme der Flussdichte im Nahbereich des Kreuzungspunktes, so dass eine relativ starke Magnetfeldkonzentration auf eine kleine Körperfläche ermöglicht wird. Auf diese Weise lassen sich stark lokalisierte Erkrankungen, wie zum Beispiel Abszesse und Infekte, wirkungsvoll behandeln.

Die Erfindung ist in besonders vorteilhafter Weise dadurch weitergebildet, dass der Funktionsstromgenerator geeignet ist, in Abhängigkeit von an die Eingangsschnittstelle der Steuereinheit übertragenen Signalen quasi rein-harmonische Spannungsverläufe mit einem ersten Oberwellenanteil oder entartet-harmonische Spannungsverläufe mit einem zweiten Oberwellenanteil, der größer als der erste Oberwellenanteil ist, zu erzeugen. Durch die Veränderung des von dem Funktionsstromgenerator erzeugten Spannungsverlaufs können die Oberwellenanteile des elektromagnetischen Feldes im erkrankten Körperbereich verändert werden. Insbesondere kann dabei die Frequenz des niederfrequenten Magnetfeldes aufrechterhalten werden, während aber elektrische Felder, welche vom zeitlichen Differential des Magnetfeldes abhängen, bis hin zu hohen Frequenzen variiert werden können. Man kann auf diese Weise den erkrankten Körperbereich also quasi unveränderlichen niederfrequenten magnetischen Wechselfeldem aussetzen, die die Differenzierung der Zellen begünstigen, während hochfrequente Anteile dann erzeugt werden, wenn dies aufgrund verminderter Induktionsspannungen sinnvoll ist.

Konkret ist im Falle der Stimulierung im Bereich von Implantaten vorgesehen, dass der Funktionsstromgenerator geeignet ist, bei Vorliegen einer durch die Erfassungseinrichtung erfassten Spannung, die einer Sollspannung entspricht oder in einem Sollspannungsintervall liegt, quasi rein-harmonische Spannungsverläufe zu erzeugen und bei einem Unterschreiten der Sollspannung oder des Sollspannungsintervalls entartet-harmonische Spannungsverläufe zu erzeugen.

In diesem Zusammenhang ist insbesondere nützlich, dass der Funktionsstromgenerator in eine Regelung eingebunden ist, die durch Veränderung der Spannungsverläufe zwischen rein-harmonisch und entartet-harmonisch die von der Erfassungseinrichtung erfasste Spannung auf eine Sollspannung oder in ein Sollspannungsintervall regelt.

Eine besonders bevorzugte Ausgestaltung der Vorrichtung besteht darin, dass mehrere Implantate, jeweils zugehörige Übertragerspulenanordnungen, jeweils zugehörige Elektrodenpaare, jeweils zugehörige Erfassungseinrichtungen und jeweils zugehörige Übertragungseinrichtungen vorgesehen sind, dass der Funktionsstromgenerator auf der Grundlage der geringsten oder der geringsten noch über einem Mindestspannungswert liegenden von einer der Erfassungseinrichtungen gemessenen Spannung beeinflussbar ist und dass den Elektrodenpaaren Spannungsbegrenzer zugeordnet sind. Will man in jedem der Implantate, deren Übertrager mitunter unterschiedliche Winkel zur Magnetfeldrichtung einnehmen, die gewünschte physiologisch besonders gut wirksame Spannung von beispielsweise 700mV erzeugen, so ist es erforderlich, den Schwingungsverlauf des erregenden Magnetfeldes unter Berücksichtigung der am ungünstigsten zum Magnetfeld stehenden Übertragerspule zu beeinflussen. Da dies zur Folge hat, dass ohne geeignete Gegenmaßnahmen die Spannungswerte an anderen, günstiger zum Magnetfeld stehenden Übertragern überhöht wären, wird dies durch den einzelnen Übertragern zugeordnete Spannungsbegrenzer vermieden. Zeigen bestimmte Übertrager eine sehr geringe Spannung, so könnte es aber auch sinnvoll sein, diese unberücksichtigt zu lassen und erst solche Übertragerspannungen zur Grundlage der Schwingungsbeeinflussung zu machen, die oberhalb von einem bestimmten Schwellenwert, beispielsweise 200 mV oder 300 mV, liegen.

Die vorliegende Erfindung betrifft eine Vorrichtung zum Stimulieren eines Heilungsprozesses. Die Erfindung ließe sich aber auch als Verfahren formulieren, bei dem es wesentlich auf die folgenden Verfahrensschritte ankommt: Erzeugen eines elektromagnetischen Feldes im Bereich eines Implantates, das mit einer Obertragerspulenanordnung gekoppelt ist, wobei die Pole der Übertragerspulenanordnung mit einem Elektrodenpaar verbunden sind; erfassen einer von der Übertragerspulenanordnung erzeugten Spannung; Übertragen von für die elektrische Spannung charakteristischen Signalen an eine Steuereinheit eines Funktionsstromgenerators; Beeinflussen eines von dem Funktionsstromgenerator erzeugten Spannungsverlauf in Abhängigkeit der übertragenen Signale. Besonders vorteilhafte Ausführungsformen dieses Verfahrens sind dadurch gekennzeichnet, dass die Übertragung der Informationen an die Steuereinheit unter Verwendung von mindestens einem RFID-Transponder erfolgt. Weiterhin ist das Verfahren besonders dann vorteilhaft, wenn der Funktionsstromgenerator in eine Regelung eingebunden ist, die durch Veränderung der Spannungsverläufe zwischen rein-harmonisch und entartet-harmonisch die erfasste Spannung auf eine Sollspannung oder in ein Sollspannungsintervall regelt.

Die Erfindung wird nun mit Bezug auf die begleitenden Zeichnungen anhand besonders bevorzugter Ausführungsformen beispielhaft erläutert.

Es zeigen:
- Figur 1: eine erfindungsgemäße Vorrichtung während ihrer Anwendung;
- Figur 2: ein der erfindungsgemäßen Vorrichtung zugehöriges Implantat;
- Figur 3: einen rein-harmonischen Spannungsverlauf;
- Figur 4: einen entartet-harmonischen Spannungsverlauf;
- Figur 5: eine in einer erfindungsgemäßen Vorrichtung einsetzbare Spulenanordnung in einem ersten Zustand;
- Figur 6: eine in einer erfindungsgemäßen Vorrichtung einsetzbare Spulenanordnung in einem zweiten Zustand;
- Figur 7: eine schematische Darstellung zur Erläuterung der räumlichen Ausrichtung eines durch eine erfindungsgemäße Spulenanordnung erzeugten Magnetfeldes
- Figur 8: eine geschnittene perspektivische Teildarstellung einer Spulenanordnung mit plastischen Eigenschaften und
- Figur 9: eine Anordnung mit mehreren Implantatkomponenten zur Erläuterung eines weiteren Aspektes der Erfindung.

Bei der nachfolgenden Beschreibung der Zeichnungen bezeichnen gleiche Bezugszeichen gleiche oder vergleichbare Komponenten.

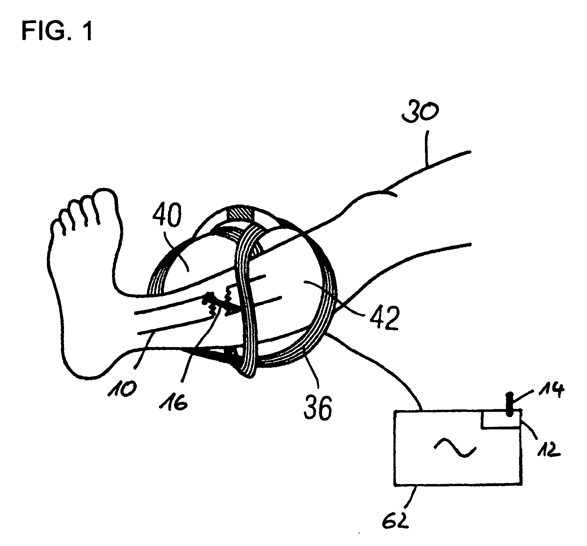

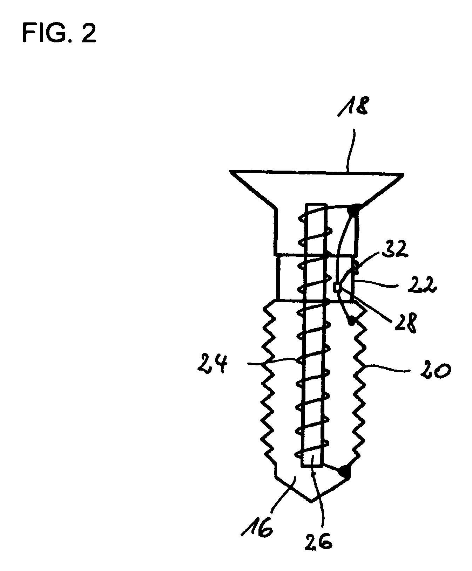

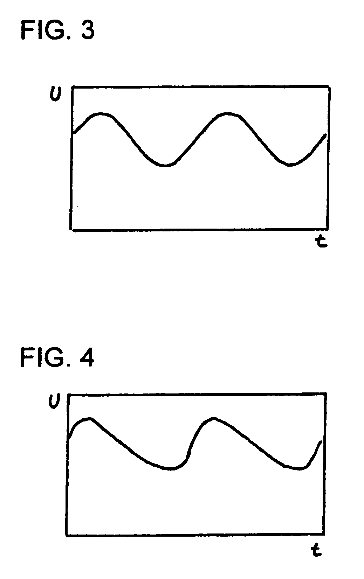

Figur 1 zeigt eine erfindungsgemäße Vorrichtung während ihrer Anwendung. Figur 2 zeigt ein der erfindungsgemäßen Vorrichtung zugehöriges Implantat. Die Figuren 3 und 4 zeigen Signalverläufe, die von der erfindungsgemäßen Vorrichtung erzeugt werden können. In Figur 1 ist ein Bein 30 mit einem gebrochenen Unterschenkelknochen 10 zu erkennen. Das induzierende elektromagnetische Feld wird in einer flexiblen Magnetspule 36 von einem Funktionsstromgenerator 62 erzeugt. Zur besseren Darstellung wurde die flexible Spule 36 mit einem Abstand zum erkrankten Körperbereich dargestellt. In der Praxis kann die Magnetspule 36 aufgrund ihrer Flexibilität unmittelbar an den Körper beziehungsweise einen Verband angeformt werden, so dass ihre Wicklung den lädierten Bereich in geringem Abstand umfängt.

Die Fragmente des Unterschenkelknochens sind durch ein Implantat 16 miteinander verbunden und werden durch dieses gestützt. Bei dem Implantat 16 kann es sich insbesondere um eine Knochenschraube handeln, in oder an der die für die Stimulation erforderlichen Komponenten angeordnet sind. Eine solche Knochenschraube 16 ist in Figur 2 im Querschnitt dargestellt. Die Knochenschraube 16 weist einen elektrisch leitfähigen proximalen Bereich mit Schraubenkopf 18, einen mit einem Gewinde 20 ausgestatteten distalen Bereich und einen zwischen dem proximalen und dem distalen Bereich angeordneten isolierenden Bereich 22 auf. Die durch den isolierenden Bereich 22 getrennten elektrisch leitfähigen Bereiche sind mit den Polen einer Übertragerspule 24 verbunden, die einen weichmagnetischen Kern 26 aufweist. Weiterhin steht eine Erfassungseinrichtung 28 mit den Polen der Übertragerspule 24 mittelbar oder unmittelbar in elektrisch leitfähiger Verbindung. Ebenso ist ein RFID-Transponder 32 vorgesehen, der über eine Leitung mit der Erfassungseinrichtung 28 gekoppelt ist. Auf diese Weise kann die in dem RFID-Transponder 32 gespeicherte Information in Abhängigkeit von der in der Übertragerspule 24 induzierten Spannung verändert werden, so dass letztlich über ein Lesegerät 14 der dem Funktionsstromgenerator 62 zugeordneten Steuereinheit 12 ein Maß für die induzierte Spannung ermittelt werden kann. Um das Auslesen des RFID-Transponders 32 problemlos zu ermöglichen, ist dieser im vorliegenden Ausführungsbeispiel außerhalb der Knochenschraube 16, also nicht innerhalb der Knochenschraube 16, angeordnet. Hierdurch wird eine Abschirmung der Hochfrequenzfelder, die zum Auslesen des RFID-Transponders 32 benötigt werden, vermieden. Es ist aber auch denkbar, den RFID-Transponder 32 an geeigneter Stelle innerhalb der Schraube anzuordnen, insbesondere innerhalb des isolierenden Bereichs 22. Mit der vorliegenden Anordnung gelingt es also, die induzierte elektrische Spannung U_{ind} = dB/dt mittelbar oder unmittelbar zu messen und auf eine vorzugsweise einen Mikroprozessor aufweisende Steuereinheit im Funktionsstromgenerator der induzierenden Magnetspule zu übertragen. Die Steuereinheit 12, der die Steuerung des Funktionsstromes der induzierenden Magnetspule obliegt, reagiert auf das RFID-Signal mit einer Änderung der Signalform (dB/dt) vom zeitlich symmetrischen Sinus-Verlauf zu einem zeitlich unsymmetrischen Verlauf seiner ansteigenden beziehungsweise abfallenden Flanke des Stromes der induzierenden Magnetspule. Figur 3 zeigt die unmodulierte Grundschwingung, während Figur 4 eine modulierte Schwingung mit aufgesteilten ansteigenden Flanken zeigt. Durch Vergleich mit der durch eine zeitlich symmetrische oberwellenfreie Sinusschwingung induzierten Spannung, die beispielsweise um einen Betrag von 200 mV kleiner ist als die Sollspannung, wird die Steilheit (dB/dt) einer Flanke der Sinusschwingung erhöht, bis der Sollwert U_{ind} = 700 mV am Ausgang der Aufnehmerspule erreicht ist. Dies kann im Rahmen einer Regelung erfolgen. Jede der in den Figuren 3 und 4 gezeigten Signalformen erfüllt dabei die Anforderungen, Oberwellen der Sinus-Grundschwingung nur bis maximal 1 kHz, vorzugsweise bis 500 Hz, bei einer maximalen Flussdichte von 3 mT zu erzeugen und die Resonanzfrequenz zellulärer Botenstoffe als Grundschwingung aufzuweisen. Um eine störungsfreie Übertragung der hochfrequenten elektromagnetischen Signale zwischen dem RFID-Transponder 18 und der als Lesegerät 14 ausgebildeten Eingangsschnittstelle der Steuereinheit 12 zu gewährleisten, kann es erforderlich sein, die Erzeugung elektromagnetischer Felder in der Magnetspule 36 zum Zwecke der Kommunikation für kurze Zeit auszusetzen. Hier zeigt sich die besondere Stärke der Verwendung von RFID-Transponder im Vergleich zu einem herkömmlichen Sender, der aktiv Signale in Abhängigkeit der erzeugten Spannung aussenden kann. Der oder die verwendeten RFID-Transponder arbeiten nämlich aufgrund von gespeicherten Informationen, so dass es nicht erforderlich ist, das erregende Magnetfeld aufrechtzuerhalten, um Aufschluss über die induzierte Spannung zu erhalten.

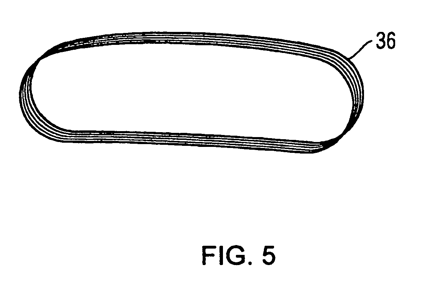

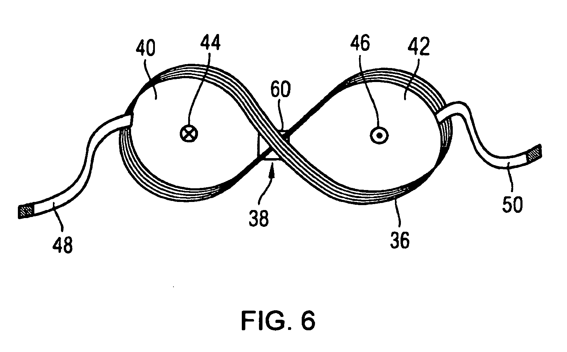

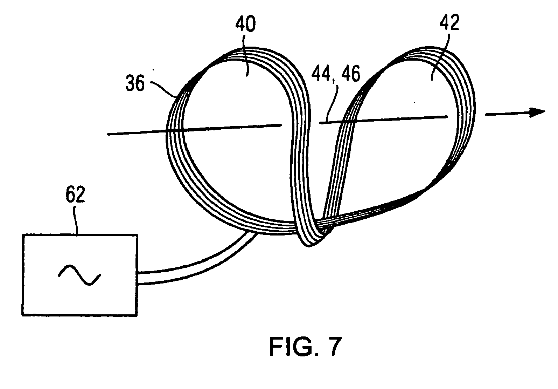

Figur 5 zeigt eine in einer erfindungsgemäßen Vorrichtung einsetzbare Spulenanordnung in einem ersten Zustand. Figur 6 zeigt eine in einer erfindungsgemäßen Vorrichtung einsetzbare Spulenanordnung in einem zweiten Zustand. Figur 7 zeigt eine schematische Darstellung zur Erläuterung der räumlichen Ausrichtung eines durch eine erfindungsgemäße Spulenanordnung erzeugten Magnetfeldes. Nutzt man die Flexibilität der Spulenanordnung 36 dazu aus, diese in eine achtförmige Gestalt zu bringen, so werden zwei Flächen 40, 42 sowie ein Kreuzungspunkt 38 definiert. Der Kreuzungspunkt 38 ist durch eine Kopplungseinrichtung 60 fixiert und kann vorzugsweise verschoben werden, so dass das Verhältnis der Flächen 40, 42 variabel ist. Beispielsweise kann als Kopplungseinrichtung 60 ein elastisches Band mit Klettverschluss oder Gürtelschließe dienen. Ebenfalls ist es denkbar, Oberflächen der Spulenanordnung so auszustatten, dass sie die Komponenten eines Klettverschlusses bilden und auf diese Weise an verschiedenen Stellen direkt aufeinander haften können. Um die Einstellung zu erleichtern, können im Variationsbereich Vermerke oder Skalen vorgesehen sein; an diesen kann sich der Anwender orientieren, wenn er eine bestimmte Fixierung des Kreuzungspunktes 38 vornehmen möchte. Fließt durch eine solche Spulenanordnung 36 ein Wechselstrom, so haben die induzierten Magnetfelder entgegengesetzte Richtungen, wie durch die Vektorsymbole 44, 46 angedeutet ist. Aufgrund der Flexibilität der Spulenanordnung 36 kann diese aber auch in eine andere Gestalt gebracht werden. Dies ist im Zusammenhang mit Figur 1 dargestellt, wo auch ein Bein mit einem gebrochenen Unterschenkelknochen 10 als Beispiel eines erkrankten Körperbereichs gezeigt ist. Nun liegen sich die Flächen 40, 42 gegenüber und die in den jeweiligen Segmenten der Spule erzeugten Magnetfelder haben die gleiche Richtung. Dies ist in Figur 7, in der auch ein mit der Spulenanordnung gekoppelter Funktionsstromgenerator 62 gezeigt ist, nochmals veranschaulicht, nämlich durch den einheitlichen Magnetfeldvektor 44, 46, der beide Flächen 40, 42 durchdringt. Gemäß Figur 1 durchdringt das Magnetfeld den erkrankten Körperbereich im Wesentlichen senkrecht zur Längsachse der Extremität. Ebenfalls ist es möglich, dass die Extremität die von den Schlaufen der achtförmigen Spule definierten Flächen durchdringt, so dass dann das Magnetfeld im Wesentlichen parallel zur Achse der Extremität verläuft.

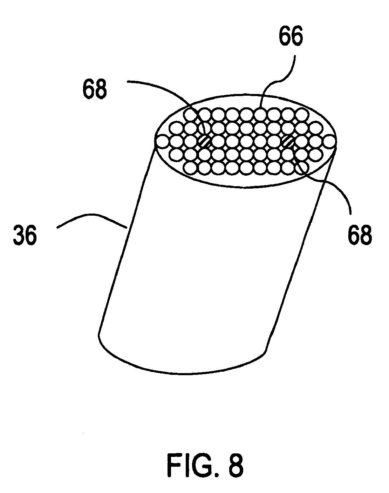

Figur 8 zeigt eine geschnittene perspektivische Teildarstellung einer Spulenanordnung mit plastischen Eigenschaften. Innerhalb der Spulenanordnung 36 ist die elektrisch leitende Spulenwicklung 66 zu erkennen. Zusätzlich sind parallel zu der Spulenwicklung 66 zwei Adern 68 aus plastischem Material vorgesehen, die der Spulenanordnung 36 insgesamt eine plastische Flexibilität vermitteln.

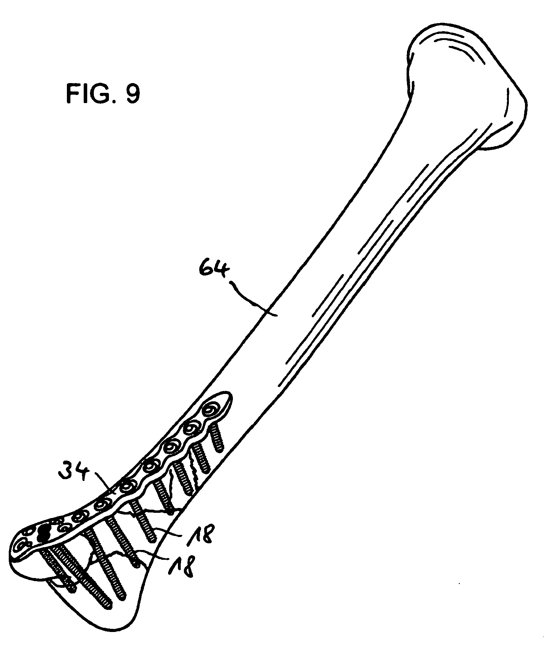

Figur 9 zeigt eine Anordnung mit mehreren Implantatkomponenten zur Erläuterung eines weiteren Aspektes der Erfindung. Bei Osteosynthesen von beispielsweise einer Trümmerfraktur durch mehrere Schrauben mit jeweils einer Aufnehmerspule sind unterschiedliche Richtungen der Aufnehmerspulen zur Richtung des induzierenden Magnetfeldes möglich und werden damit an den Schrauben unterschiedliche elektrische Spannungen aktiviert. In Figur 9 ist dies am Beispiel einer durch Schrauben 18 am Knochen 64 festgelegten Knochenstützplatte 34 dargestellt. Die Regelung im Sinne einer Zunahme der induzierten elektrischen Spannung (U_{ind} = dB/dt) soll deshalb solange erfolgen, bis alle Aufnehmerspulen der Knochenschrauben - unabhängig von Ihrer Richtung zur der des induzierenden EMF - die Sollspannung von 700 mV erreicht haben. Zum Schutz vor unerwünschter Induktion von mehr als 700 mV wird der Ausgang jeder Aufnehmerspule mit einer Diode von 700 mV Sperrspannung überbrückt.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste:

- 10: Erkrankter Körperbereich
- 12: Steuereinheit
- 14: Eingangsschnittstelle / Lesegerät
- 16: Knochenschraube / Implantat
- 18: Schraubenkopf
- 20: Gewinde
- 22: Isolierender Bereich
- 24: Übertragerspule / Übertragerspulenandordnung
- 26: Weichmagnetischer Kern
- 28: Erfassungseinrichtung
- 30: Bein
- 32: RFID-Transponder / Übertragungseinrichtung / Sender
- 34: Knochenplatte
- 36: Spulenanordnung / Magnetspule / Spule
- 38: Kreuzungspunkt
- 40: Fläche
- 42: Fläche
- 44: Magnetfeld / Magnetfeldvektor / Vektorsymbol
- 46: Magnetfeld / Magnetfeldvektor / Vektorsymbol
- 60: Kopplungseinrichtung
- 62: Funktionsstromgenerator
- 64: Knochen
- 66: Spulenwicklung
- 68: Plastische Ader

## Patentansprüche

1. Vorrichtung zum Stimulieren eines Heilungsprozesses mit
- einer mit einem Funktionsstromgenerator (62) gekoppelten Spulenanordnung (36) zum Erzeugen eines elektromagnetischen Feldes in einem erkrankten Körperbereich (10),
- einer Steuereinheit (12) zum Beeinflussen eines von dem Funktionsstromgenerator (62) erzeugten Spannungsverlaufs in Abhängigkeit von an eine Eingangsschnittstelle (14) der Steuereinheit übertragenen Signalen,
- mindestens einem in dem erkrankten Körperbereich anordenbaren Implantat (16), das mit einer Übertragerspulenanordnung (24) gekoppelt ist, wobei die Pole der Übertragerspulenanordnung mit einem Elektrodenpaar verbunden sind,
**gekennzeichnet durch**
- eine Erfassungseinrichtung (28) zum Erfassen einer zwischen den Elektroden des Elektrodenpaars vorliegenden elektrischen Spannung,
- eine Übertragungseinrichtung (32) zum Übertragen von für die elektrische Spannung charakteristischen Signalen an die Steuereinheit.

2. Vorrichtung nach Anspruch 1, **dadurch gekennzeichnet, dass** die Übertragungseinrichtung mindestens einen Sender (32) zur drahtlosen Kommunikation mit einem der Eingangsschnittstelle der Steuereinheit (12) zugehörigen Empfänger (14) aufweist.

3. Vorrichtung nach Anspruch 1 oder 2, **dadurch gekennzeichnet, dass** die Übertragungseinrichtung mindestens einen RFID-Transponder (32) aufweist, dessen Informationsgehalt von einem der Eingangsschnittstelle der Steuereinheit (12) zugehörigen Lesegerät (14) erfassbar ist.

4. Vorrichtung nach Anspruch 3, **dadurch gekennzeichnet, dass** der auslesbare Informationsgehalt des RFID-Transporiders (32) in Abhängigkeit von Signalen, die von der Erfassungseinrichtung (28) geliefert werden, veränderbar ist.

5. Vorrichtung nach Anspruch 3 oder 4, **dadurch gekennzeichnet, dass** mehrere RFID-Transponder vorgesehen sind, die in Abhängigkeit von Signalen, die von der Erfassungseinrichtung geliefert werden, aktivierbar oder deaktivierbar sind.

6. Vorrichtung nach einem der vorangehenden Ansprüche, **dadurch gekennzeichnet, dass** die Spulenanordnung eine Spule mit einer einen Kreuzungspunkt (38) aufweisenden Spulenwicklung aufweist, die durch eine achtförmige Gestalt zwei Flächen definiert (40, 42), wobei die Flächen (40, 42) so zueinander ausgerichtet sind, dass die von einem durch einen Stromfluss in der Spulenanordnung (36) erzeugten, die Flächen durchdringenden Magnetfelder (44, 46) im Wesentlichen gleichgerichtet sind.

7. Vorrichtung nach Anspruch 6, **dadurch gekennzeichnet, dass** die Spulenanordnung (36) flexibel ist, so dass die Flächen (40, 42) auf gegenüberliegenden Seiten des erkrankten Körperbereichs (10) anordenbar sind.

8. Vorrichtung nach einem der vorangehenden Ansprüche, **dadurch gekennzeichnet, dass** der Funktionsstromgenerator (62) geeignet ist, in Abhängigkeit von an die Eingangsschnittstelle (14) der Steuereinheit (12) übertragenen Signalen quasi rein-harmonische Spannungsverläufe mit einem ersten Oberwellenanteil oder entartet-harmonische Spannungsverläufe mit einem zweiten Oberwellenanteil, der größer als der erste Oberwellenanteil ist, zu erzeugen.

9. Vorrichtung nach Anspruch 8, **dadurch gekennzeichnet, dass** der Funktionsstromgenerator (62) geeignet ist, bei Vorliegen einer durch die Erfassungseinrichtung (28) erfassten Spannung, die einer Sollspannung entspricht oder in einem Sollspannungsintervall liegt, quasi rein-harmonische Spannungsverläufe zu erzeugen und bei einem Unterschreiten der Sollspannung oder des Sollspannunsintervalls entartet-harmonische Spannungsverläufe zu erzeugen.

10. Vorrichtung nach Anspruch 9, **dadurch gekennzeichnet, dass** der Funktionsstromgenerator (62) in eine Regelung eingebunden ist, die durch Veränderung der Spannungsverläufe zwischen rein-harmonisch und entartet-harmonisch die von der Erfassungseinrichtung (28) erfasste Spannung auf eine Sollspannung oder in ein Sollspannungsintervall regelt.

11. Vorrichtung nach einem der vorangehenden Ansprüche, **dadurch gekennzeichnet, dass** mehrere Implantate, jeweils zugehörige Übertragerspulenanordnungen, jeweils zugehörige Elektrodenpaare, jeweils zugehörige Erfassungseinrichtungen und jeweils zugehörige Übertragungseinrichtungen vorgesehen sind, dass der Funktionsstromgenerator auf der Grundlage der geringsten oder der geringsten noch über einem Mindestspannungswert liegenden von einer der Erfassungseinrichtungen gemessenen Spannung beeinflussbar ist und dass den Elektrodenpaaren Spannungsbegrenzer zugeordnet sind.

## Claims

1. Apparatus for stimulating a healing process comprising
- a coil arrangement (36) coupled to a functional power generator (62) for generating an electromagnetic field in an affected body region (10),
- a control unit (12) for influencing a voltage curve generated by the functional power generator (62) dependent on signals transmitted to an input interface (14) of the control unit,
- at least one implant (16) positioned in the affected body region and coupled to a transformer coil arrangement (24), the poles of the transformer coil arrangement being connected to a pair of electrodes,
**characterized by**
- a sensing device (28) for detecting an electric voltage existing between the electrodes of the pair of electrodes,
- a transmission device (32) for transmitting signals characteristic of the electric voltage to the control unit.

2. Apparatus according to claim 1, **characterised in that** the transmission device comprises at least one transmitter (32) for a wireless communication with a receiver (14) associated with the input interface of the control unit (12).

3. Apparatus according to claim 1 or 2, **characterised in that** the transmission device comprises at least one RFID transponder (32) the information content of which is detectable by a reading device (14) associated with the input interface of the control unit (12).

4. Apparatus according to claim 3, **characterised in that** the readable information content of the RFID transponder (32) is changeable depending on signals supplied by the sensing device (28).

5. Apparatus according to claim 3 or 4, **characterised in that** a plurality of RFID transponders is provided which can be activated or deactivated depending on signals supplied by the sensing device.

6. Apparatus according to one of the preceding claims, **characterised in that** the coil arrangement comprises a coil comprising a coil winding having an intersection point (38) which defines two surfaces (40, 42) by means of a figure eight shaped form, the surfaces (40, 42) being aligned with respect to each other so that the magnetic fields (44, 46) generated by a current flow in the coil arrangement (36) and pervading the surfaces are substantially oriented in a same direction.

7. Apparatus according to claim 6, **characterised in that** the coil arrangement (36) is flexible so that the surfaces (40, 42) can be positioned at opposite sides of the affected body region (10).

8. Apparatus according to one of the preceding claims, **characterised in that** the functional power generator (62) is capable of generating virtually purely harmonic voltage curves having a first harmonic wave component or abnormally harmonic voltage curves having a second harmonic wave component which is greater than the first harmonic wave component depending on signals transmitted to the input interface (14) of the control unit (12).

9. Apparatus according to claim 8, **characterised in that** the functional power generator (62) is capable of generating virtually purely harmonic voltage curves in the presence of a voltage detected by the sensing device (28) which corresponds to a target voltage or is within a target voltage interval, and of generating abnormally harmonic voltage curves when the voltage falls below the target voltage or the target voltage interval.

10. Apparatus according to claim 9, **characterised in that** the functional power generator (62) is integrated in a control that regulates the voltage detected by the sensing device (28) to a target voltage or a target voltage interval by changing the voltage curves between purely harmonic and abnormally harmonic.

11. Apparatus according to one of the preceding claims, **characterised in that** a plurality of implants, corresponding transformer coil arrangements, corresponding pairs of electrodes, corresponding sensing devices and corresponding transmission devices are provided, that the functional power generator can be influenced on the basis of the lowest voltage measured by one of the sensing devices or the lowest voltage measured by one of the sensing devices which is still higher than a minimum voltage value, and that voltage limiters are allocated to the pairs of electrodes.

## Revendications

1. Dispositif pour stimuler un processus de guérison, comportant:
- un agencement de bobine (36) couplé à un générateur de courant fonctionnel (62) pour générer un champ électromagnétique dans une partie atteinte du corps (10),
- une unité de contrôle (12) pour influer une courbe de tension générée par le générateur de courant fonctionnel (62) en fonction de signaux transmis à une interface d'entrée (14) de l'unité de contrôle,
- au moins un implant (16) qui peut être disposé dans la partie du corps atteinte et qui est couplé à un agencement de bobine transductrice (24), les pôles de l'agencement de bobine transductrice étant reliés à une paire d'électrodes,
**caractérisé par**
- un dispositif de détection (28) pour détecter une tension existante entre les électrodes de la paire d'électrodes,
- un dispositif de transmission (32) pour transmettre des signaux caractéristiques de la tension électrique à l'unité de contrôle.

2. Dispositif selon la revendication 1, **caractérisé en ce que** le dispositif de transmission comporte au moins un émetteur (32) pour une communication sans fil avec un récepteur (14) associé à l'interface d'entrée de l'unité de contrôle (12).

3. Dispositif selon les revendications 1 ou 2, **caractérisé en ce que** l'unité de transmission comporte au moins un transpondeur RFID (32), le contenu informatif duquel est détectable par un dispositif de lecture (14) associé à l'interface d'entrée de l'unité de contrôle (12).

4. Dispositif selon la revendication 3, **caractérisé en ce que** le contenu informatif lisible du transpondeur RFID (32) peut être changé en fonction de signaux fournis par le dispositif de détection (28).

5. Dispositif selon les revendications 3 ou 4, **caractérisé en ce que** plusieurs transpondeurs RFID qui sont activables ou déactivables en fonction de signaux fournis par le dispositif de détection sont prévus.

6. Dispositif selon l'une des revendications précédentes, **caractérisé en ce que** l'agencement de bobine comporte une bobine ayant un bobinage comportant un point de croisement (38) qui définit deux surfaces (40, 42) par une forme en forme de huit, les surfaces (40, 42) étant orientées l'une vers l'autre de sorte que les champs magnétiques générés par un flux de courant dans l'agencement de bobine (36) et traversant les surfaces sont orientés substantiellement pareil.

7. Dispositif selon la revendication 6, **caractérisé en ce que** l'agencement de bobine (36) est flexible de sorte que les surfaces (40, 42) peuvent être disposées sur des côtés opposés de la partie du corps atteinte (10).

8. Dispositif selon l'une des revendications précédentes, **caractérisé en ce que** le générateur de courant fonctionnel (62) est capable de générer des courbes de tension quasi purement harmoniques avec une première composante d'onde harmonique ou des courbes de tension harmoniques dégénérées avec une deuxième composante d'onde harmonique supérieure à la première composante d'onde harmonique en fonction de signaux transmis à l'interface d'entrée (14) de l'unité de contrôle (12).

9. Dispositif selon la revendication 8, **caractérisé en ce que** le générateur de courant fonctionnel (62) est capable de générer des courbes de tension quasi purement harmoniques en présence d'une tension détectée par le dispositif de détection (28) qui correspond à une tension nominale ou qui est dans un intervalle de tension nominal, et de générer des courbes de tension harmoniques dégénérées lorsque ladite tension est inférieure à la tension nominale ou l'intervalle de tension nominal.

10. Dispositif selon la revendication 9, **caractérisé en ce que** le générateur de courant fonctionnel (62) est intégré dans un réglage qui règle la tension détectée par le dispositif de détection (28) sur une tension nominale ou dans un intervalle de tension nominal par variation des courbes de tension entre purement harmonique et harmonique dégénérée.

11. Dispositif selon l'une des revendications précédentes, **caractérisé en ce que** plusieurs implants, des agencements de bobine transductrices correspondants, des paires d'électrodes correspondantes, des dispositifs de détection correspondants et des dispositifs de transmission correspondants sont prévus, **en ce que** le générateur de courant fonctionnel peut être influé sur la base de la tension minimale mesurée par l'un des dispositifs de détection ou sur la base de la tension minimale mesurée par l'un des dispositifs de détection qui reste supérieure à une valeur de tension minimale, et **en ce que** des limiteurs de tension sont associés aux paires d'électrodes.
